**Europäisches Patentamt**

**European Patent Office**

**Office européen des brevets**

(19)

(11) Veröffentlichungsnummer: **0 151 121 B1**

# EUROPÄISCHE PATENTSCHRIFT

(12)

(45) Veröffentlichungstag der Patentschrift:
07.10.87

(21) Anmeldenummer: **84901608.4**

(22) Anmeldetag: **10.04.84**

(86) Internationale Anmeldenummer:
**PCT/HU 84/00022**

(87) Internationale Veröffentlichungsnummer:
**WO 84/04211 (25.10.84 Gazette 84/25)**

(51) Int. Cl.⁴: **H 01 S 3/17,** H 01 S 3/092, H 01 S 3/11, H 01 S 3/045, A 61 N 5/06

(54) **FLÜSSIGKEITSGEKÜHLTER NEODYM-PHOSPHATGLAS-IMPULSLASER INSBESONDERE ZU MEDIZINISCHEN ANWENDUNGEN.**

(30) Priorität: **11.04.83 HU 124289**

(43) Veröffentlichungstag der Anmeldung:
**14.08.85 Patentblatt 85/33**

(45) Bekanntmachung des Hinweises auf die Patenterteilung:
**07.10.87 Patentblatt 87/41**

(84) Benannte Vertragsstaaten:
**AT BE CH DE FR GB LI LU NL SE**

(56) Entgegenhaltungen:
CH - A - 612 800
DE - A - 2 436 623
DE - A - 3 112 284
GB - A - 1 383 139
GB - A - 1 424 251
US - A - 3 633 126
US - A - 4 227 159

WISSENSPEICHER LASERTECHNIK, 1st edition 1982, VEB Fachbuchverlag Leipzig, pages 138,145,146,152-161,233-236,260,305

(73) Patentinhaber: **MAGYAR TUDOMANYOS AKADEMIA KÖZPONTI FIZIKAI KUTATO INTEZETE, Konkoly Thege ut, Budapest XII (HU)**

(72) Erfinder: **CSERY, Huba, Mihályfy E.u. 26/B, H-1022 Budapest (HU)**
Erfinder: **CZIGANY, Imre, Kardhegy u. 15., H-1116 Budapest (HU)**
Erfinder: **HORVATH, Zoltán, Galgóczy köz 7/A, H-1125 Budapest (HU)**
Erfinder: **KERTESZ, Iván, Koltö u. 2-4, H-1121 Budapest (HU)**
Erfinder: **KROO, Norbert, Apáczai Cs.J.u. 17., H-1052 Budapest (HU)**
Erfinder: **SCHMIDT, György, u. 38., H-1066 Budapest (HU)**

(74) Vertreter: **Lehn, Werner, Dipl.-Ing. et al, Hoffmann, Eitle & Partner Patentanwälte Arabellastrasse 4, D-8000 München 81 (DE)**

## Beschreibung

Die Erfindung betrifft einen flüssigkeitsgekühlten Neodym-Phosphatglas-Impulslaser mit passiver Q-Schaltung in tragbarer Ausführung für Anwendung vor allem in der Medizin, insbesondere in der Augenchirurgie, ferner in Spektroskopie, Kosmetic, im Unterricht und in der Mikrotechnologie.

Bei der Augentherapie ist die Anwendung von Photokoagulatoren bekannt. Früher wurden Xenonlampen-Einrichtungen angewendet, deren lange Expositionszeit (250-1000 ms) eine spezielle Betäubung erfordert. Ausserdem ist in den meisten Fällen der auf Retina erreichbare fokussierte Lichtfleckendurchmesser (500-1000 μm) zu gross. Zur Beseitigung dieser Nachteile wurden die Laserfotokoagulatoren erarbeitet, womit es möglich ist, das Lichtbündel des Lasers auf eine bedeutend kleinere Fläche zu konzentrieren, im optimalen Fall mit einem Durchmesser kleiner als 10 μm. Daher ist die erwünschte biologische Wirkung bei um Grössenordnungen kleineren Impulslängen erreichbar, so wird eine spezielle Betäubung überflüssig, es genügt die Fixierung des Kopfes des Patienten und die Anwendung von Mitteln zur Pupillenerweiterung. Für die erfolgreiche Behandlung genügt schon 1 bis 5% der Energie des Lichtimpulses der Xe-Lampe, oft sogar noch eine wesentlich kleinere Energie.

In der früheren Phase der Anwendung wurden in erster Linie die im Impulsbetrieb arbeitenden Rubinlaser verwendet, heute dagegen sind in breiten Kreisen die mit ionisiertem Ar oder Kr ausgebildeten Gaslaser kontinuierlichen Betriebs verbreitet. Bei diesen sind die Expositionszeiten verhältnismässig lang, in der Grössenordnung von 0,1 s, aber ihr Licht ist relativ gut fokussierbar. Ihr Nachteil: weil die Expositionszeit im Verhältnis zur Betriebsdauer kurz ist, wird die von dem Laser ausgestrahlte Lichtenergie nur zu einem kleinen Teil genutzt.

Da die Energie der zur Behandlung benutzten Laserimpulse — und damit auch die nicht erwünschten Nebenwirkungen — durch die Reduzierung der Impulslänge verringert werden können, zeigt sich in den letzten zwei Jahren anwachsendes Interesse an den Festkörperlasern, in erster Linie an den Q-geschalteten, bzw. an den modensynchronisierten Nd:YAG-Lasern. Die Masse dieser Geräte sind indessen sehr gross, so ist ihre Anwendung wesentlich nur in feststehender Ausführung möglich, da neben dem beträchtlichen Gewicht auch ihre Stromaufnahme sehr hoch ist und im allgemeinen eine Kühlung von der Wasserleitung erfordert. Informationen über diese Systeme sind in den Zeitschriften LASER FOCUS (Februar 1983) und LASERS AND APPLICATIONS (October 1982) zu finden.

Das Ziel der vorliegenden Erfindung ist die Verwirklichung eines Lasers, der die oben genannten Nachteile ausschliesst und die Schaffung eines tragbaren, an das normale Einphasennetz anschliessbaren, mit geschlossenem Kühlkreislauf ausgestatteten Gerätes, das breite Anwendung insbesondere in der Augentherapie finden kann.

Der Erfindung liegt die Erkenntnis zugrunde, dass in das Lithiumphosphatglas in hoher Konzentration Nd-Ionen eingebracht werden können, mit denen auf der Wellenlänge von 1,054 μm um ein 2-3 mal höherer Wirkungsgrad der Laserfunktion mit einer typisch 3-5 mal kleineren Bündeldivergenz erreichbar ist, als beim Nd:YAG-Laser. Alles das ist durch einen Laserstab von kleinen Abmessungen — mit insbesondere einem Durchmesser von 3 mm und einer Länge von 55 mm verwirklichbar.

Unter Verwendung der erwähnten Erkenntnis wurde der eingangs erwähnte flüssigkeitsgekühlte Neodym-Phosphatglas-Impulslaser erfindungsgemäss so ausgestattet, dass er einen in einem Resonator angeordneten, ohne Verstellen des Resonators abmontierbaren, flüssigkeitsgekühlten Laserkopf, der eine Blitzlampe und einen Laserstab mechanisch fixiert, einen geschlossenen Kühlkreislauf, eine an ein Einphasennetz anschliessbare Speiseeinheit und insbesondere eine Elektronik zur Anzeige der Betriebsart aufweist, dass der im Resonator angeordnete passive Q-Schalter als ein mit $F_2$-Zentren gefärbter LiF-Kristall mit einer passiven Transmission von insbesondere 70% ausgebildet ist, dass der Resonator einen hochreflektierenden Sperrspiegel und einen Austrittsspiegel mit einer Transmission von insbesondere 60% enthält, und dass das aktive Material des Laserstabs aus Phosphatglas mit einer Nd-Ionen-Konzentration im Bereich von $10^{21}$ Ionen/$cm^3$ besteht. Insbesondere beträgt die Nd-Ionen-Konzentration 1,2 bis $1,4 \cdot 10^{21}$ Ionen/$cm^3$.

Der erfindungsgemässe Laser ermöglicht es, Impulse im $TEM_{oo}$-Modus mit unter 20 ns liegender Impulslänge, kleiner als 3 mrad ausmachender Winkeldivergenz, bis zu 50 mJ erhöhbarer Energie und einer maximalen Wiederholungsfrequenz von 8 Hz zu erzeugen, wobei die Wellenlänge der kohärenten Lichtimpulse 1,054 μm ist.

Die Wiederholungsfrequenz der Laserimpulse ist bis zu den maximalen Werten kontinuierlich veränderbar. Die Kühlung des Laserkopfes erfolgt mit Kühlflüssigkeit im geschlossenen Kreislauf. Das Gerät kann an das normale Einphasennetz angeschlossen werden, seine Leistungsaufnahme ist typisch 50 W, seine Gesamtmasse ca. 2 kg.

Besondere Ausführungsarten der Erfindung sollen ferner folgende Gesichtspunkte berücksichtigen:

— Der Laser soll von kleinen Abmessungen, leicht transportierbar, von geringem Gewicht, auf die vorhandenen Einrichtungen leicht montierbar sein.

— Der Laser soll eine flexible Verbindung mit der Speiseeinheit und dem Kühlsystem haben, wobei diese Verbindung auch die elektrischen und Kühlleitungen beeinhaltet. Deshalb muss eine Stromversorgung mit relativ niedriger Spannung gesichert sein, damit die Berührungsschutzanforderungen eingehalten werden.

— Die tragbare, überall aufstellbare Ausführung verlangt ausser dem Anspruch auf eine Netzspannung insbesondere 220 V (die meisten medizinischen Laser arbeiten mit Dreiphasenspannung), auch ein geschlossenes Kühlsystem, so wird sie unabhängig von der zentralen Wasserleitung.

— Im Falle der bei den Festkörperlasern üblichen Lösung erscheint die Spannung sofort am Anfang des langzeitigen Aufladens an der Blitzlampe, so dass sich diese wegen der kleinen elektrischen transienten Störungen auch zufällig entladen kann. Dies hätte unabsehbare Folgen bei medizinischen Eingriffen. Um das zu verhindern, ist eine solche Speiseeinheit zu entwickeln, bei der ohne Startsignal keine Spannung an der Blitzlampe anliegt, so ist die Möglichkeit eines zufälligen Laserimpulses ausgeschlossen, da nämlich die Anfladezeit so kurz ist, dass das Aufladen und der Laserimpuls praktisch gleichzeitig mit dem Startsignal erfolgt, und der Laser bis zum folgenden Startsignal ohne Spannung bleibt.

Es ist vorteilhaft, wenn eine als Pumpquelle angewandte Blitzlampe und der Laserstab in einem von aussen mit einer reflektierenden Schicht überzogenen und von einer Kühlflüssigkeit, insbesondere von destilliertem Wasser, durchströmten Glasrohr angeordnet sind.

Die gute Austauschbarkeit der pumpenden Blitzlampe kann erreichbar sein, wenn der Resonator als eine ohne Verstellung abnehmbare Einheit ausgebildet ist.

Da die Einrichtung vom laserphysikalischen Standpunkt aus gesehen für die Anwendung durch Laien bestimmt ist, und diese, wegen der Alterung der Blitzlampe ziemlich labile Betriebsart des Festkörperlasers wiederholtes Nachstellen erfordert, wurde die Einbaumöglichkeit einer Überprüfungselektronik als Option offen gelassen, die auf einfache Art anzeigt, welche Korrektur mit dem Regelelement zum optimalen Betrieb vorgenommen werden muss.

Ebenso bleibt bei der Grundkonstruktion die Möglichkeit eines späteren Einbaus von Pilotlicht, Fehleranzeigen, Laserimpulszähler, Frequenzverdopper und Filterkombinationen, Energiemesser offen. Diese Einheiten können auch den bekannten Prinzipien nach ausgebildet werden.

Auf Grund der Abmessungen und der Kosten wählten wir eine Blitzlampe, die nach ca. 50 000 Blitzen (bei normaler ärztlicher Praxis ca. 2-4 Wochen Betrieb) auszuwechseln ist. Die einfache Durchführung des Lampenwechsels erfordert aber ebenfalls eine vom üblichen abweichende Laserkonstruktion: In diesem Falle kann zum Lampenwechsel der Laserkopf, ohne dass sich der Resonator verstellt, einfach aus dem Laser herausgehoben und ebenso einfach wieder zurückplaciert werden.

Nachfolgend wird ein Ausführungsbeispiel der Erfindung unter Bezugnahme auf die anliegenden Zeichnungen im einzelnen erläutert. Es zeigen:

Fig. 1 Eine schematische Darstellung des Lasers mit Kühlsystem und Speiseeinheit gemäss der Erfindung;

Fig. 2 Aufbau des Lasers;

Fig. 3 Schema des Kühlkreislaufes;

Fig. 4 Blockschema der Speiseeinheit.

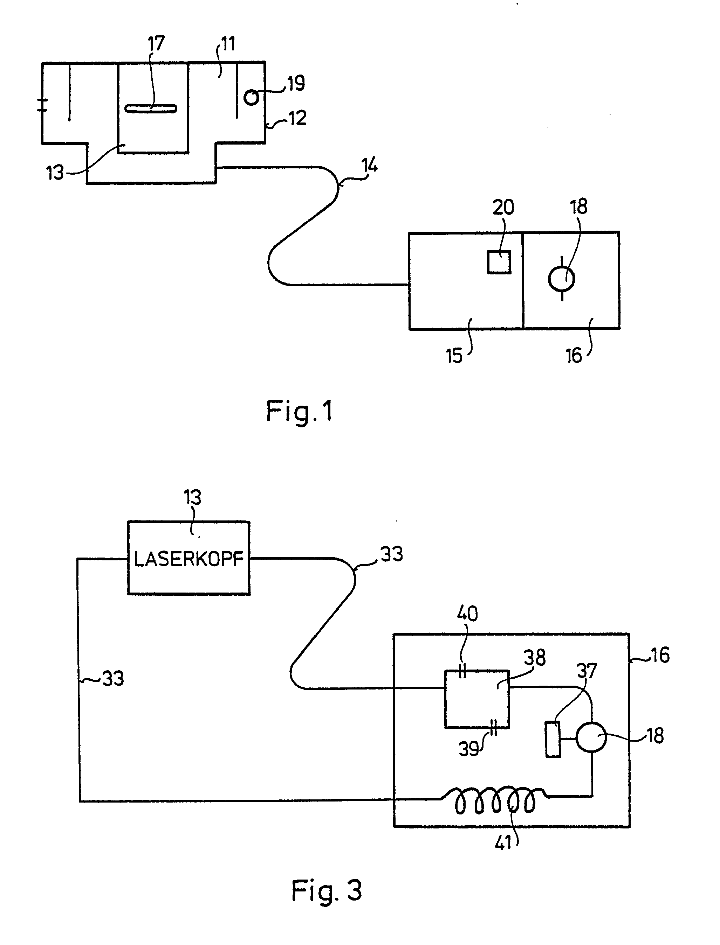

Die Hauptbestandteile des Gerätes sind in Figur 1 zu sehen. Ein Laser 11, der einen Resonator 12 und einen leicht auswechselbaren Laserkopf 13 enthält, ist durch ein für die sichere Leitung von Kühlflüssigkeit und elektrischem Strom geeignetes, in einem flexibelen Rohr untergebrachtes Leitungsbündel 14 mit einer Speiseeinheit 15 verbunden, die mit einem Kühlsystem 16 zusammengebaut ist.

Die Speiseeinheit 15 versorgt mit Energie eine Blitzlampe 17, eine Kreislaufpumpe 18, einen den Laserimpuls anzeigenden Photodetektor 19 und, wenn erforderlich, eine Elektronik 20.

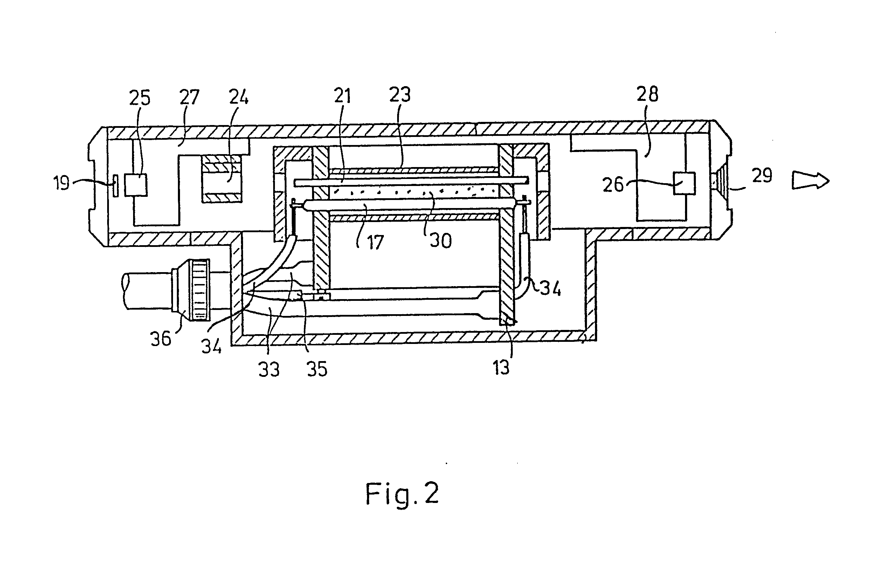

Der Aufbau des Lasers 11 ist in Fig. 2 zu sehen Einen dem Laser 11 als aktives Element dienender Laserstab 21 mit einem 2-4 mm, insbesondere 3 mm, Durchmesser und Blitzlampe 17 umgibt ein aus Glasrohr hergestellter Reflektor 23, dessen äussere Oberfläche versilbert und zum Schutz gegen Beschädigung mit einer weiteren durchsichtigen Lackschicht abgedeckt ist. Der Laserkopf 13 fixiert mechanisch die Blitzlampe 17, den Laserstab 21 und den Reflektor 23 und sichert den Zu- und Abfluss der im Glasrohr des Reflektors 23 strömenden Kühlflüssigkeit. Die Kühlflüssigkeit gelangt durch elastische Rohre 33 zum Laserkopf 13. Mit diesen gemeinsam werden in den Laserkopf 13 über eine Anschlussschraube 36 Kabel 34 der Speisespannung der Blitzlampe 17 und ein Kabel 35 des Zündimpulses geführt.

Ein hochreflektierender Sperrspiegel 25 und ein teilweise reflektierender Austrittsspiegel 26 eines aus Planspiegeln ausgebildeten Laserresonators sind in mit Differentialschrauben justierbaren Spiegelhaltern 27, 28 von Lammellenstruktur befestigt. Die Transmission des Austrittsspiegels 26 liegt zwischen 55-65% insbesondere bei 60%. Ein aus LiF-Kristall gefertigter Q-Schalter 24 ist an dem Spiegelhalter 27 des Sperrspiegels 25 befestigt. Der Q-Schalter 24 ist dicker als 1 mm, zweckmässig wenigstens so dick, wie der Durchmesser des Laserstabes 21, seine Transmission beträgt 65-75%, insbesondere 70%. Der Laserstrahl tritt aus einer entsprechender Öffnung 29 des Resonators aus, und der Photodetektor 19 des Betriebsanzeigers detektiert die durch den Sperrspiegel 25 durchgehenden schwachen Lichtimpulse.

Wenn der Laser 11 auf den Impuls der Blitzlampe 17 nicht reagiert, registriert der Photodetektor 19 keinen Lichtimpuls, bei normalem Funktionieren registriert er einen, bei fehlerhaftem Funktionieren zwei Impulse.

Im Falle der richtigen Betriebsart — deren Einstellung durch die Anzeige der Betriebsart unterstützt wird — bekommt man also einen Impuls.

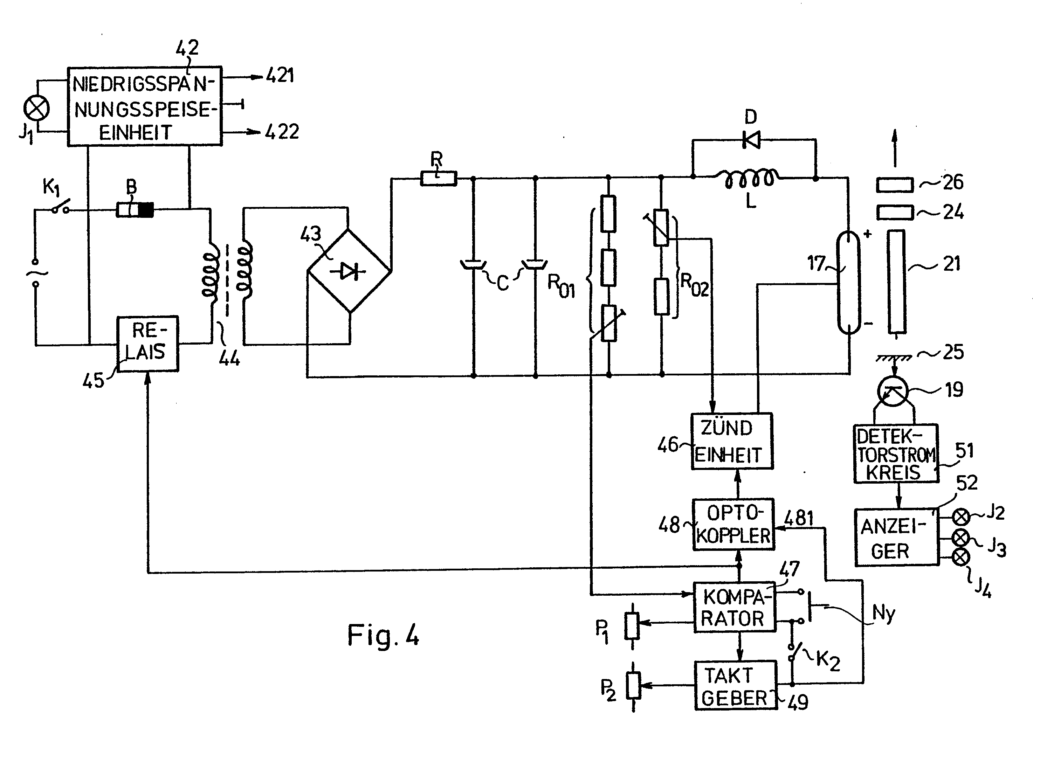

Die Figur 3 zeigt den schematischen Aufbau des Kühlsystems 16, das ausser dem Laserkopf 13 und den elastischen Rohren 33 aus der Kreislaufpumpe 18 und einem sie antreibenden Motor 37, einem Wärmeaustauscher 41, einem Puffergefäss 38 der Kühlflüssigkeit und Ventilen 39, 40 des Abflusses bzw. Zuflusses besteht. Die Kühlflüssigkeit ist üblicherweise schwach basisches destilliertes Wasser, dessen Basizität insbesondere mit Trinat-Hydrophosphat eingestellt wird.

Die Figur 4 zeigt das Blockschema der Speiseeinheit 15. Eine Niedrigspannungsspeiseeinheit 42 und ein Festkörperrelais 45 sind der Primärseite eines mit einem Hauptschalter $K_1$ und einer Sicherung 3 versehenen Hochspannungstransformators angeschlossen. Die Sekundärseite des Hochspannungstransformators 44 ist durch einen Gleichrichter 43, einen Widerstand R, parallel geschaltete Speicherkondensatoren C und Spannungsteiler $R_{01}$, $R_{02}$, eine von einer Diode D überbrückte Induktivität L einerseits der Blitzlampe 17, andererseits einer Zündeinheit 46 zugeführt. Der Spannungsteiler $R_{01}$ ist mit einem Komparator 47 verbunden der mit einem Ausgang an einen Optokoppler 48, sowie an das Festkörperrelais und mit einem anderen Ausgang über einen Schalter $K_2$ an einen Taktgeber 49 angeschlossen und mit einem Fussschalter Ny versehen ist. Der Taktgeber 49 ist einem Sperreingang 481 des Optokopplers 48 zugeführt und mit einem Potentiometer $P_2$ versehen. Der Komparator ist mit einem Potentiometer $P_1$ ausgestattet. Der Ausgang des Optokopplers 48 ist der Zündeinheit 46 zugeführt. Die Blitzlampe 17 ist in optischer Kopplung mit dem zwischen dem Q-Schalter (mit dem nachgeordneten Austrittsspiegel 26) und dem Sperrspiegel (mit dem nachgeordneten Photodetektor 19) eingefügten Laserstab 21. Der Photodetektor 19 ist mit einem Detektorstromkreis 51 und dadurch mit einer Anzeigeeinheit 52 mit Lampen $J_2$, $J_3$ und $J_4$ verbunden.

Die mit einer Lampe $J_1$ versehene Niedrigspannungsspeiseeinheit 42 sichert an Ausgängen 421 und 422 die Speisespannung für die Elektronik 20 und den Motor 37 für den Kreislauf. Die für die Entladung der Blitzlampe 17 nötige Elektroenergie liefern die Speicherkondensatoren C, deren Aufladen nach dem Gleichrichter 43, mit Hilfe des Hochspannungstransformators 44 geschieht. Der Widerstand R schützt die Dioden des Gleichrichters 43 vor Stromstössen am Anfang der Aufladung. Eine Induktivität L stellt die Entladungsgeschwindigkeit der Kondensatoren C durch die Blitzlampen 17, d.h. die Dauer des Blitzes, ein. Die Diode D behebt die Spannungssprünge (Transiente) an der Induktivität L. Das Aufladen wird vor den Laserimpulsen durch das Festkörperrelais 45 auf der Primärseite geschaltet. Andernfalls liegt an der Blitzlampe 17 keine Spannung an. Die Steuerung der Zündeinheit 46 erfolgt über den mit dem Sperreingang 481 versehenen Optokoppler 48 in Form von Einzelimpulsen oder Impulsserien. Die zur Blitzlampe 17 gelangten Spannung und somit auch ihre Energie kann mittels des Potentiometers $P_1$ der Komparatoreinheit 47 kontinuierlich verändert werden. Der Komparator 47 beobachtet die Spannung an den Kondensatoren C über den Spannungsteiler $R_{01}$. Die Zündspannung der Zündeinheit 46 kann mit Hilfe des Spannungsteilers $R_{02}$ eingestellt werden. Die Wiederholungsfrequenz der Laserimpulse ist durch Stellen des Potentiometers $P_2$ des Taktgebers 49 (Frequenzsteuereinheit) bis zu einer maximalen Frequenz von 8 Hz veränderbar. Die Überprüfung der Betriebsart geschieht durch das Verarbeiten der Signale des Photodetektors 19 mit Hilfe des Detektorstromkreises 51 und der Anzeigeeinheit 52 mit ihren Lampen $J_2$, $J_3$, $J_4$. Neben einem Blitzlampenimpuls leuchtet die Lampe $J_2$ bei Fehlen eines Laserimpulses, die Lampe $J_3$ bei einem, die Lampe $J_4$ bei zwei Laserimpulsen auf.

Der Laser 11 arbeitet so, dass er von aussen nur 220 V (auf Wunsch 110 V) Wechselspannung und eine Leistung von typisch 20-25 W beansprucht. Mit dem Hauptschalter $K_1$ wird das Gerät eingeschaltet, was die Lampe $J_1$ anzeigt. Damit beginnt die Niedrigspannungsspeiseeinheit 42 zu arbeiten. Mit dem Potentiometer $P_1$ wird die benötigte Energie für die Blitzlampe 17 eingestellt, mit dem Potentiometer $P_2$ die Wiederholungsfrequenz. Mit dem Ausschalten des Schalters $K_2$ und der Inbetriebnahme des Fussschalters Ny entstehen einzelne Impulse. Wird der Fussschalter Ny betätigt, so laden sich die Speicherkondensatoren C auf, und die Blitzlampe 17 bekommt Spannung. Auf die Wirkung des darauf folgenden Zündimpulses zündet die Blitzlampe 17, und wenn der Laser 11 richtig eingestellt ist, das heisst wenn der Sperrspiegel 25 und der Austrittsspiegel 26 in richtiger Position sind, und das Licht der Blitzlampe 17 genügend stark ist, zündet die Lampe $J_3$ der Anzeigeeinheit 52.

Der Laserresonator lässt sich auf jedes beliebige optische System aufmontieren, insbesondere auf Zieleinrichtungen, die bei Augenoperationen benutzt werden, z.B. auf die optische Zieleinrichtung, bzw. auf die Einrichtung zum Fixieren des Kopfes der Photokoagulatoren, die Ar- oder Kr-Ion-Laser benutzen. Nötigenfalls kann man auf die zwei Seiten des Laserresonators je eine Lampe und eine, diese Lichtquellen in Richtungen der Achse des Laserbündes fokussierende Optik, d.h. Zieleinrichtung montieren. In der Brennebene dieser Optik kann man das Licht der zwei Lichtquellen auf einen Punkt in der Achse des Laserbündels zusammenbringen.

Ein charakteristischer Vorteil des Lasers gemäss der Erfindung ist seine gute Bündelqualität ($TEM_{oo}$-Modus und kleine Winkeldivergenz). Das Bündel ist typisch auf einen Kreis mit unter 10 μm liegendem Diameter fokussierbar.

Das Anwendungsgebiet des Gerätes liegt im allgemeinen in der Augenchirurgie, aber es ist auch anzuwenden bei Geräten der Sepktroskopie als Anregungslichtquelle, bei Behandlungen mit Akupunktur, in der Kosmetik und in der Mikrotechnologie (Schweissen, Justieren, usw.).

**Patentanprüche**

1. Flüssigkeitsgekühlter Neodym-Phosphatglas-Impulslaser mit einem passiven Q-Schalter in tragbarer Ausführung für Anwendungen vor allem in der Medizin, insbesondere in der Augenchirurgie, ferner in der Spektroskopie, Kosmetik, im Unterricht und in der Mikrotechnologie, dadurch gekennzeichnet, dass er einen in einem Resonator angeordneten, ohne Verstellen des Resonators abmontierbaren, flüssigkeitsgekühlten Laserkopf (13), der eine Blitzlampe (17) und einen Laserstab mechanisch fixiert, einen geschlossenen Kühlkreislauf (16), eine an ein Einphasennetz anschliessbare Speiseeinheit (15) und insbesondere eine Elektronik zur Anzeige der Betriebsart aufweist, dass der im Resonator angeordnete passive Q-Schalter als ein mit $F_2$-Zentren gefärbter LiF-Kristall (24) mit einer passiven Transmission von insbesondere 70% ausgebildet ist, dass der Resonator einen hochreflektierenden Sperrspiegel (25) und einen Austrittsspiegel (26) mit einer Transmission von insbesondere 60% enthält, und dass das aktive Material des Laserstabs (21) aus Phosphatglas mit einer Nd-Ionen-Konzentration im Bereich von $10^{21}$ Ionen/cm³ besteht.

2. Neodym-Phosphatglas-Impulslaser nach Anspruch 1, dadurch gekennzeichnet, dass die als Pumpquelle angewandte Blitzlampe (17) und der Laserstab (21) in einem von aussen mit einer reflektierenden Schicht überzogenen und von einer Kühlflüssigkeit insbesondere von destilliertem Wasser, durchströmten Glasrohr (23) angeordnet sind.

3. Neodym-Phosphatglas-Impulslaser nach Anspruch 1 oder 2, dadurch gekennzeichnet, dass der Resonator als eine, ohne Verstellung abnehmbare Einheit ausgebildet ist.

4. Neodym-Phosphatglas-Impulslaser nach einem der Ansprüche 1 oder 3, dadurch gekennzeichnet, dass die Kühlung des die Blitzlampe (17) und des aktiven Material enthaltenden Laserkopfes (13) durch zirkulierende Kühlflüssigkeit gesichert wird, die im geschlossenen Kreislauf durch ein elastisches Rohr (33) fliesst und von einer durch einen kleinen Elektromotor (37) angetriebenen Punpe (18) in Bewegung gebracht wird, und die ihre im Laserkpf (13) aufgenommene Wärmeenergie in einem Flüssigkeit-Luft Wärmeaustauscher (41) abgibt.

5. Neodym-Phosphatglas-Impulslaser nach einem der Ansprüche 1 bis 4, dadurch gekennzeichnet, dass die die Blitzlampe (17) des Laserkopfes antreibende Speiseeinheit (15) mit einer Energie von max. 20 J entweder einzelne, mit einem Fussschalter auslösbare Impulse oder mit einer Frequenz bis maximal 8 Hz kontinuierlich einstellbare periodische Impulse abgeben kann, die Speiseeinheit (15) der Blitzlampe (17) gegen Überspannung gesichert ist, und die Blitzlampe bei fehlendem Startsignal ohne Spannung ist.

6. Neodym-Phosphatglas-Impulslaser nach Anspruch 1 oder 2, dadurch gekennzeichnet, dass der Laserresonator auf die Zieloptik von Photokoagulatoren für Augenheilkunde mit einem Ar-Ion-Laser aufmontiert werden kann.

7. Neodym-Phosphatglas-Impulslaser nach einem der Ansprüche 1 bis 6, dadurch gekennzeichnet, dass die Haltemechanik des Laserresonators zum einen mit den Spiegeln (25, 26) und dem Q-Schalter (24), zum anderen mit dem wassergekühlten Laserkopf (13) unabhängig voneinander verbunden ist.

8. Neodym-Phosphatglas-Impulslaser nach einem der Ansprüche 1 bis 7, dadurch gekennzeichnet, dass die Spiegel (25, 26) mit ihren Halterungen mit Hilfe von Differentialschrauben fein justierbar sind.

9. Neodym-Phosphatglas-Impulslaser nach einem der Ansprüche 1 bis 8, dadurch gekennzeichnet, dass in dem Laserkopf (13) die Kühlung der Blitzlampe (17) und des Laserstabes (21) von einer Kühlflüssigkeit gesichert wird, die in einem die Blitzlampe (17) und den Laserstab (21) umgebenden, von aussen versilberten und danach mit einer durchsichtigen Lackschicht überzogenen zylindrischen Glasrohr (23) strömt.

10. Neodym-Phosphatglas-Impulslaser nach einem der Ansprüche 1 bis 9, dadurch gekennzeichnet, dass er einen Frequenzverdoppler-Kristall enthält, welcher mit angeschlossenen speziellen Filtern im austretenden Bündel die Mischung von Infrarotlicht der Wellenlänge 1,054 μm und grünem Licht der Wellenlänge 0,527 μm in beliebigem Verhältnis möglich macht.

11. Neodym-Phosphatglas-Impulslaser nach einem der Ansprüche 1 bis 10, dadurch gekennzeichnet, dass der Phosphatglasssstab (21) mit einem

Durchmesser von 2 bis 4 mm, inbesondere von 3 mm, ausgebildet ist und die Konzentration der Nd-Ionen insbesondere $1,2 \cdot 10^{21}$ Ionen/cm$^3$ beträgt.

## Claims

1. Fluid-cooled neodyme phosphate glass impulse laser with a passive «Q» switch as in a portable arrangement for use mainly in medicine, particularly for eye surgery, in spectroscopy, cosmetics, in teaching and in micro-technology characterized in that it includes:
   a fluid cooled laser head (13) arranged in a resonator, which can be disassembled without de-tuning of the resonator, said laser head (13) mechanically fixing a flash lamp (17) and a laser bar;
   a closed cooling circuit (16);
   a supply unit (15) which can be connected to a single phase supply; and
   especially an electronic circuit for displaying the operation mode;
   in that the passive «Q» switch arranged in the resonator is formed as a $F_2$-centre-coloured LiF-crystal (24) with a passive transmission of particularly 70%;
   in that the resonator includes a highly reflecting blocking mirror (25) and an output mirror (26) with a transmission of particularly 60%;
   and in that the active material of the laser bar (21) consists of phosphate glass with a Nd-ion concentration in the range of $10^{21}$ ions per cm$^3$.

2. Neodyme phosphate impulse laser according to claim 1 characterized in that the flash lamp (17) used as a pumping source and the laser bar (21) are arranged in a glass tube (23) covered with a reflecting layer on the outside, through which a cooling liquid, in particular distilled water, flows.

3. Neodyme phosphate impulse laser according to claim 1 o 2, characterized in that the resonator is formed as a unit which can be removed without detuning.

4. Neodyme phosphate glass impulse laser according one of the claims 1 to 3 characterized in that the cooling of the flash lamp (17) and of the laser head (13) comprising the active material is ensured by the circulating cooling fluid which flows through an elastic tube (33) in the closed cooling circuit and is set in motion by a pump (18) driven by a small electric motor (37) and which releases its heat energy absorbed from the laser head (13) in a fluid air heat exchanger (41).

5. Neodyme phosphate glass impulse laser according to one of the claims 1 to 4 characterized in that the supply unit (15) which drives the flash lamp (17) of the laser head can supply either individual impulses triggered by a foot switch or periodic impulses with a frequency of up to a maximum of 8Hz which can be continually adjusted, said impulses having an energy of 20J maximum, said supply unit (15) of the flash lamp (17) being protected against over voltages, and said flash lamp is without voltage when a start signal is lacking.

6. Neodyme phosphate glass impulse laser according to claim 1 or 2 characterized in that the laser resonator can be mounted onto the target optics of photo-coagulators for eye therapy using an Ar-ion laser.

7. Neodyme phosphate glass impulse laser according to one of the claims 1 to 6 characterized in that the holding mechanism of the laser resonator is independently of each other on the one hand connected with the mirrors (25, 26) and the «Q» switch (24) and on the other hand with the water cooled laser head (13).

8. Neodyme phosphate glass impulse laser according to one of the claims 1 to 7 characterized in that the mirrors (25, 26) with their holders are finely adjustable with the help of differential screws.

9. Neodyme phosphate glass impulse laser according to one of the claims 1 to 8 characterized in that the cooling of the flash lamp (17) and the laser bar (21) in the laser head (13) is ensured by a cooling fluid, which flows through a cylindrical glass tube (23) silvered on the outside and afterwards covered with a transparent varnish layer, and which surrounds the flash lamp (17) and the laser bar (21).

10. Neodyme phosphate glass impulse laser according to one of the claims 1 to 9 characterized in that it includes a frequency doubling crystal, which when connected with special filters allows the mixture of infra-red light of the wave length 1.054 microns and green light of the wave length 0.527 microns in the emerging beam in any desired ratio.

11. Neodyme phosphate glass impulse laser according to one of the claims 1 to 10 characterized in that the phosphate glass bar (21) is formed with a diameter of 2 to 4 mm, in particular of 3 mm and the concentration of the Nd-ions is in particular $1.2 \times 10^{21}$ ions per $cm^3$.

## Revendications

1. Laser à impulsions à verre au phosphate de néodyme refroidi par un fluide avec un interrupteur Q passif, réalisé en version portative, pricipalement prévu pour des applications médicales, notamment pour la chirurgie des yeux et, en outre, en spectroscopie, en cosmétologie, dans l'enseignement et la microtechnologie, caractérisé en ce qu'il comprend une tête de laser (13) disposée dans un résonateur et susceptible d'être démontée sans déréglage du résonateur et refroidie par un fluide, cette tête de laser fixant une lampe-éclair (17) et une barre laser mécaniquement, un circuit de refroidissement fermé (16), une unité d'alimentation (15) raccordable à un réseau monophasé et, en particulier une électronique de signalisation du mode de fonctionnement, en ce que l'interrupteur Q passif disposé dans le résonateur est sous forme d'un cristal LiF (24) teinté avec des centres $F_2$ et avec une transmission passive qui est notamment de 70%, en ce que le résonateur comporte un miroir d'interception (25) à taux de réflexion élevé et un miroir de sortie (26) dont le taux de transmission est en notamment de 60%, et en ce que la matière active de la barre laser (21) est constituée par un verre au phosphate d'une concentration ionique en Nd de l'ordre de $10^{21}$ ions/$cm^3$.

2. Laser à impulsions à verre au phosphate de néodyme d'après la revendication 1, caractérisé en ce que la lampe-éclair (17) utilisée comme source de pompage et la barre laser (21) sont disposées dans un tube de verre (23) revêtu extérieurement d'une couche réfléchissante et parcouru par un fluide de refroidissement, notamment de l'eau distillée.

3. Laser à impulsions à verre au phosphate de néodyme, d'après la revendication 1 ou 2, caractérisé en ce que le résonateur est réalisé comme une unité amovible sans réglage.

4. Laser à impulsions à verre au phosphate de néodyme d'aprés une des revendications 1 à 3, caractérisé en ce que le refroidissement de la tête de laser (13) qui contient la lampe-éclair (17) et la matière active est assurée par la circulation du liquide de refroidissement qui s'écoule en circuit fermé dans un tube élastique (33) et est mis en mouvement par une pompe (18) entraînée par un petit moteur électrique (37), tandis que l'énergie calorifique absorbée dans la tête de laser (13) est cédée au sein d'un échangeur de chaleur air-liquide (41).

5. Laser à impulsions à verre au phosphate de néodyme d'après une des revendications 1 à 4, caractérisé en ce que l'umidité d'alimentation (15) qui actionne la lampe-éclair (17) de la tête de laser peut émettre avec une énergie maximum de 20 J soit des impulsions individuelles déclenchables au moyen d'un interrupteur à pédale, soit des impulsions périodiques réglables en continu d'une fréquence maximale de 8 Hz, en ce que l'unité d'alimentation (15) de la lampe-éclair (17) est protégée contre les surtensions et en ce que la lampe-éclair est privée de tension en l'absence d'un signal de départ.

6. Laser à impulsions à verre au phosphate de néodyme d'après la revendication 1 ou 2, caractérisé en ce que le résonateur de laser peut être monté sur l'équipement optique de visée de photocoagulateurs d'ophtalmologie avec un laser à ions d'Ar.

7. Laser à impulsions à verre au phosphate de néodyme d'après une des revendications 1 à 6, caractérisé en ce que la mécanique d'arrêt du résonateur de laser est reliée aux miroirs (25, 26) et à l'interrupteur Q (24) d'une part, et à la tête du laser (13) refroidie par eau, ces deux liaisons étant indépendantes.

8. Laser à impulsions à verre au phosphate de néodyme d'après une des revendications 1 à 7, caractérisé en ce que les miroirs (25, 26) peuvent être ajustés avec précision avec leurs fixations à de vis différentielles.

9. Laser à impulsions à verre au phosphate de néodyme d'après une des revendications 1 à 8, caractérisé en ce que dans la tête de laser (13) le refroidissement de la lampe-éclair (17) et de la barre laser (21) est assuré par un fluide de refroidissement qui s'écoule dans un tube de verre cylindrique (23) extérieurement argenté et recouvert par dessus d'une couche de vernis transparent, qui entoure la lampe-éclair (17) et la barre laser (21).

10. Laser à impulsions à verre au phosphate de néodyme d'après une des revendications 1 à 9, caractérisé en ce qu'il contient un cristal doubleur de fréquence qui permet, au moyen de filtres spéciaux raccordés, d'opérer sur le faisceau sortant le mélange de lumiére infrarouge de 1, 054 µm de longueur d'onde et de lumière verte de 0,527 µm de longueur d'onde selon un rapport facultatif.

11. Laser à impulsions à verre au phosphate de néodyme d'après une des revendications 1 à 10, caractérisé en ce que la barre de verre au phosphate (21) a un diamètre de 2 à 4 mm, notamment de 3 mm, et en ce que la concentration d'ions Nd est notamment de l'ordre de $1,2 \cdot 10^{21}$ ions/cm$^3$.

Fig.1

Fig. 3

Fig. 2

0 151 121

Fig. 4

NIEDRIGSSPAN-
NUNGSSPEISE-
EINHEIT

42

→ 421
→ 422

$J_1$

$K_1$

B

RE-
LAIS

45

44

43

R

D

L

C

$R_{01}$

$R_{02}$

17

26

24

21

25

19

ZÜND
EINHEIT

46

DETEK-
TORSTROM
KREIS

51

OPTO-
KOPPLER

48

481

ANZEI-
GER

52

$J_2$
$J_3$
$J_4$

KOMPA-
RATOR

47

$P_1$

Ny

$K_2$

TAKT
GEBER

49

$P_2$

0 151 121